# EUROPÄISCHE PATENTSCHRIFT

(11) **EP 4 094 796 B1**
(45) Veröffentlichungstag und Bekanntmachung des Hinweises auf die Patenterteilung: **12.03.2025**
(21) Anmeldenummer: 22173764.6
(22) Anmeldetag: 17.05.2022
(51) Int. Cl.: A61M 16/06

(54) **BÄNDERUNG MIT BRILLENBEREICH**
BANDING WITH SPECTACLE AREA
AGENCEMENT DE BRIDES POURVU DE ZONE DE LUNETTES

(30) Priorität: 27.05.2021 DE 102021002733
(43) Veröffentlichungstag der Anmeldung: 30.11.2022
(73) Patentinhaber: Löwenstein Medical Technology S.A., 2557 Luxembourg (LU)
(72) Erfinder: Eifler, Martin, 25348 Glückstadt (DE)
(74) Vertreter: Löwenstein Medical IP

(56) Entgegenhaltungen:
- WO-A1-2021/049941
- WO-A2-2012/080887
- DE-U1- 202020 001 858
- GB-A- 1 258 710
- US-A- 4 836 200
- US-A1- 2005 061 326
- US-A1- 2006 081 252
- US-A1- 2007 056 590
- US-A1- 2007 252 946
- US-A1- 2015 283 348
- US-A1- 2016 256 655

## Beschreibung

Patienteninterfaces bzw. Atemmasken werden zur Beatmung, zur Unterstützung der Atmung oder als Schutzmaske gegen Aerosole aus festen oder flüssigen Partikeln eingesetzt.

Atemmasken für die Beatmung oder Atemunterstützung bilden die Schnittstelle zwischen einem Anwender oder Patienten und einem Beatmungsgerät und müssen hohen Anforderungen an Stabilität und Sicherheit genügen und gleichzeitig einen ausreichenden Komfort für den Anwender bieten. Atemmasken werden üblicherweise über eine Bänderung am Kopf des Anwenders fixiert. Die Bänderung kann für Anwender, die eine Brille tragen zum Problem werden, da das Tragen einer Brille zusammen mit einer Atemmaske unkomfortabel bis unmöglich ist.

US 2005/061326 A1, US 2007/056590 A1, WO 2021/049941 A1, WO 2012/080887 A2, US 2015/283348 A1, US 4 836 200 A, US 2006/081252, GB 1 258 710 A und US 2016/256655 A1 offenbaren ebenfalls Bänderungen für Patienteninterfaces.

Zu unterscheiden sind Atemmasken mit Stirnstützen und Atemmasken ohne Stirnstützen. Atemmasken mit Stirnstützen machen das Tragen einer normalen Brille nahezu unmöglich, da die Stirnstütze die Anlagefläche für eine Brille blockiert. Nachdem die Atemmaske angelegt wurde ist das Auf- und Absetzen einer normalen Brille nicht mehr möglich.

Mit stirnstützenlosen Atemmasken ist das Tragen einer Brille grundsätzlich möglich. Allerdings kann es hierbei auch zu Problemen kommen, da die Bänderung in der Regel mindestens abschnittsweise über die Schläfe verläuft, über den normalerweise auch die Brillenbügel verlaufen. Stirnstützenlose Atemmasken weisen üblicherweise Versteifungsvorrichtungen in der Bänderung auf, um eine ausreichende Stabilität und einen guten Sitz der Atemmaske zu gewährleisten. Der versteifte Bereich ist dabei in der Regel zum Gesicht des Anwenders hin gepolstert, um den Tragekomfort zu erhöhen.

Der Anwender könnte versuchen, die Brille über der Bänderung zu tragen. Dies kann jedoch dazu führen, dass die Brille verformt oder verbogen wird und nicht richtig sitzt. Wenn der Anwender die Brille unter der Bänderung hindurchschiebt, kann es zu einem unangenehmen Druck kommen, da die Brillenbügel von der Bänderung gegen das Gesicht gedrückt werden. Dieses Problem verhindert ein bequemes Tragen der Brille insbesondere über einen längeren Zeitraum. Ferner kann das Tragen der Brille mit unter der Bänderung verlaufenden Brillenbügeln den Sitz der Atemmaske negativ beeinflussen und eine Leckage verursachen.

Es besteht somit Bedarf an Atemmasken mit einer Bänderung, die die gleichzeitige Verwendung einer Brille zulassen, ohne diese zu beschädigen und zugleich einen stabilen, sicheren und komfortablen Sitz der Atemmaske sicherstellen.

Gelöst wird die Aufgabe durch eine Bänderung für ein Patienteninterface, die die kennzeichnenden Merkmale des Anspruchs 1 aufweist. Die Unteransprüche betreffen vorteilhafte Ausgestaltungen der Erfindung.

Die Erfindung betrifft eine Bänderung für ein Patienteninterface. Erfindungsgemäß weist die Bänderung zumindest bereichsweise einen Brillentunnel auf, der eingerichtet und ausgebildet ist, zumindest einen Brillenbügel zumindest teilweise aufzunehmen, wobei die Bänderung ferner einen inneren Stoffstrang, eine Verstärkungsschicht, eine Polsterschicht, einen äußeren Stoffstrang und einen Schläfenabschnitt umfasst, wobei der Schläfenabschnitt zumindest bereichsweise den Brillentunnel aufweist und derjenige Abschnitt der Bänderung ist, der nach Anlage an einen Kopf eines Anwenders über einen Schläfenbereich des Kopfes verläuft, wobei die Verstärkungsschicht und/oder der äußere Stoffstrang eine vorgeformte Kontur derart aufweisen, dass die Polsterschicht und die Verstärkungsschicht beabstandet voneinander sind und den Brillentunnel ausbilden, wobei der innere Stoffstrang und die Polsterschicht im Schläfenabschnitt keine vorgeformte Kontur aufweisen.

In manchen Ausführungsformen ist die Bänderung dadurch gekennzeichnet, dass der Brillentunnel reversibel und/oder irreversibel ausgebildet ist.

In manchen Ausführungsformen ist die Bänderung dadurch gekennzeichnet, dass der reversibel ausgebildete Brillentunnel durch die Einwirkung des Brillenbügels und/oder durch einen lösbaren Zusatz zur Bänderung ausgebildet ist.

In manchen Ausführungsformen ist die Bänderung dadurch gekennzeichnet, dass der irreversibel ausgebildete Brillentunnel durch einen unlösbaren Zusatz zur Bänderung und/oder durch eine zumindest teilweise Aussparung im inneren Stoffstrang und/oder in der Verstärkungsschicht und/oder in der Polsterschicht und/oder im äußeren Stoffstrang und/oder durch eine zumindest abschnittsweise zumindest teilweise Vorformung der Bänderung ausgebildet ist.

In manchen Ausführungsformen ist die Bänderung dadurch gekennzeichnet, dass in der Polsterschicht und/oder der Verstärkungsschicht und/oder dem Stoffstrang des Schläfenabschnitts zumindest bereichsweise eine materialbedingte Schwachstelle ausgebildet ist.

In manchen Ausführungsformen ist die Bänderung dadurch gekennzeichnet, dass die Schwachstelle derart ausgebildet und eingerichtet ist, dass durch Einwirkung des Brillenbügels im Schläfenabschnitt zumindest bereichsweise der Brillentunnel reversibel ausgebildet wird.

In manchen Ausführungsformen ist die Bänderung dadurch gekennzeichnet, dass die Schwachstelle durch einen zumindest bereichsweise geringeren Härtegrad des Materials der Polsterschicht und/oder der Verstärkungsschicht und/oder des Stoffstranges ausgebildet ist.

In manchen Ausführungsformen ist die Bänderung dadurch gekennzeichnet, dass die Polsterschicht eine Dicke aufweist, die konstant ist oder zumindest bereichsweise nicht konstant ist.

In manchen Ausführungsformen ist die Bänderung dadurch gekennzeichnet, dass die Polsterschicht eine im Wesentlichen konstante Dicke aufweist und im Schläfenabschnitt eine Dicke aufweist, die zumindest bereichsweise größer ist als die Dicke der Polsterschicht.

In manchen Ausführungsformen ist die Bänderung dadurch gekennzeichnet, dass durch Einwirkung des Brillenbügels das Material der Polsterschicht derart verdrängt wird, dass der Brillentunnel reversibel ausgebildet wird.

In manchen Ausführungsformen ist die Bänderung dadurch gekennzeichnet, dass die Bänderung mindestens ein Distanzpolster umfasst.

In manchen Ausführungsformen ist die Bänderung dadurch gekennzeichnet, dass das Distanzpolster zumindest bereichsweise mit der Bänderung verbunden ist, bevorzugt zumindest bereichsweise mit dem Schläfenabschnitt.

In manchen Ausführungsformen ist die Bänderung dadurch gekennzeichnet, dass das Distanzpolster lösbar und/oder unlösbar mit der Bänderung verbunden ist.

In manchen Ausführungsformen ist die Bänderung dadurch gekennzeichnet, dass das Distanzpolster auf der Bänderung befestigt ist und/oder in die Bänderung integriert ist.

In manchen Ausführungsformen ist die Bänderung dadurch gekennzeichnet, dass das Distanzpolster im Schläfenabschnitt derart angeordnet ist, dass der Brillentunnel reversibel und/oder irreversibel ausgebildet wird.

In manchen Ausführungsformen ist die Bänderung dadurch gekennzeichnet, dass der Brillentunnel durch eine zumindest teilweise Aussparung in der Polsterschicht und/oder in der Verstärkungsschicht und/oder in dem Stoffstrang ausgebildet ist.

In manchen Ausführungsformen ist die Bänderung dadurch gekennzeichnet, dass der Brillentunnel durch eine zumindest teilweise Aussparung der Polsterschicht und/oder der Verstärkungsschicht und/oder des Stoffstranges ausgebildet ist.

In manchen Ausführungsformen ist die Bänderung dadurch gekennzeichnet, dass der Schläfenabschnitt eine Länge L aufweist und die Aussparung in der Polsterschicht und/oder in der Verstärkungsschicht und/oder in dem Stoffstrang und/oder die Aussparung der Polsterschicht und/oder der Verstärkungsschicht und/oder des Stoffstranges 20 % bis 100 % der Länge L des Schläfenabschnitts, bevorzugt über 50 % bis 100 %, besonders bevorzugt 80 % der Länge L des Schläfenabschnitts umfasst.

In manchen Ausführungsformen ist die Bänderung dadurch gekennzeichnet, dass die Bänderung zumindest abschnittsweise zumindest teilweise eine vorgeformte Kontur aufweist, die den Brillentunnel irreversibel ausbildet.

In manchen Ausführungsformen ist die Bänderung dadurch gekennzeichnet, dass der innere Stoffstrang und/oder die Polsterschicht und/oder die Verstärkungsschicht und/oder der äußere Stoffstrang im Schläfenabschnitt eine vorgeformte Kontur aufweisen.

In manchen Ausführungsformen ist die Bänderung dadurch gekennzeichnet, dass die Bänderung im Schläfenabschnitt derart vorgeformt ist, dass die Bänderung nach Anlage am Kopf eines Anwenders zumindest teilweise derart beabstandet vom Kopf des Anwenders ist, dass der Brillentunnel ausgebildet ist.

In manchen Ausführungsformen ist die Bänderung dadurch gekennzeichnet, dass der Brillentunnel zwischen 3 mm und 30 mm hoch ausgebildet ist, bevorzugt zwischen 5 mm und 20 mm.

In manchen Ausführungsformen ist die Bänderung dadurch gekennzeichnet, dass der Brillentunnel durch die vorgeformte Kontur zwischen 3 mm und 30 mm hoch ausgebildet ist, bevorzugt zwischen 5 mm und 20 mm.

In den Figuren sind Ausführungsbeispiele der erfindungsgemäßen Bänderung 10 dargestellt. Es zeigen:
Fig. 1 beispielhaft eine Übersicht über ein System mit einem Patienteninterface mit Bänderung, das über eine Leitung mit einem Beatmungsgerät gasleitend verbunden werden kann.
Fig. 2 einen menschlichen Kopf in Seitenansicht zur Veranschaulichung von Messpunkten, anhand derer ein Schläfenbereich definiert wird.
Fig. 3 beispielhaft, wie stirnstützenlose Patienteninterfaces über eine erfindungsgemäße Bänderung am Kopf des Anwenders befestigt werden können.
Fig. 4 eine perspektivische Draufsicht auf einen Teilbereich eines Seitenabschnitts oder eines zweiten Seitenabschnitts oder eines Basisabschnitts einer erfindungsgemäßen Bänderung.
Fig. 5 einen Querschnitt durch die Bänderung im Bereich des Seitenabschnitts oder des zweiten Seitenabschnitts oder des Basisabschnitts außerhalb des Schläfenabschnitts.
Fig. 6 bis 10 verschiedene Ausführungsbeispiele des Schläfenabschnitts der Bänderung im Querschnitt, wobei Fig. 10|B eine beispielhafte Ausführungsform der Erfindung darstellt.

### Ausführungsbeispiele

In den nachfolgenden Ausführungsbeispielen ist eine Bänderung 10 gezeigt, Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

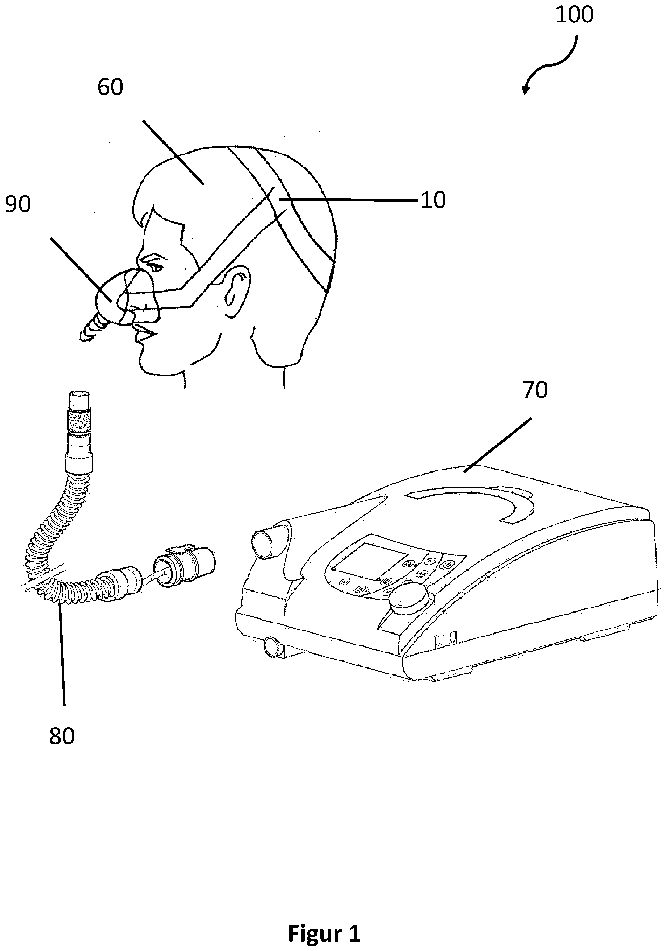

Die erfindungsgemäße Bänderung 10 eignet sich beispielsweise für die Verwendung mit einem System 100 zur Beatmung, also für die Verwendung mit einem Patienteninterface 90 und einem Beatmungsgerät 70. **Fig. 1** zeigt beispielhaft eine Übersicht über ein System 100 mit einem Patienteninterface 90 mit Bänderung 10, das über eine Leitung 80 mit einem Beatmungsgerät 70 gasleitend verbunden werden kann.

Als Patienteninterface 90 ist jegliches Peripheriegerät zu verstehen, welches zur Interaktion mit einem Lebewesen ausgebildet ist. Insbesondere ist das Patienteninterface 90 zu Therapie- oder Diagnosezwecken in Verbindung mit dem Beatmungsgerät 70 ausgebildet. Das Patienteninterface 90 kann als Atemmaske ausgebildet sein. Diese Maske kann eine Full-Face-Maske, also Nase und Mund umschließend sein, oder eine Nasenmaske, also eine nur die Nase umschließende Maske. Auch Trachealtuben bzw. -kanülen und sogenannte Nasenbrillen können als Patienteninterface 90 eingesetzt werden. Die erfindungsgemäße Bänderung 10 eignet sich insbesondere auch für Nasenpillow-Masken. Patienteninterface 90 und Atemmaske werden hierin als Synonyme verwendet. Das Patienteninterface 90 kann über eine erfindungsgemäße Bänderung 10 an den Kopf 60 eines Anwenders oder Patienten angelegt bzw. befestigt werden. Anwender und Patient werden hierin gleichbedeutend verwendet. Wenn der Begriff Anwender oder Patient genannt wird, ist jegliches Individuum gemeint, dass eine Atemmaske 90 mit Bänderung 10 verwendet.

Unter einem Beatmungsgerät 70 sind sämtliche Geräte zu verstehen, die einen Anwender oder Patienten bei der natürlichen Atmung unterstützen und/oder die Beatmung eines Anwenders oder Patienten übernehmen und/oder einer Atemtherapie dienen und/oder anderweitig auf die Atmung eines Anwenders oder Patienten einwirken. Darunter fallen zum Beispiel, aber nicht ausschließlich, CPAP- sowie BiLevel-Geräte, Narkose- bzw. Anästhesiegeräte, Atemtherapiegeräte, klinische, außerklinische oder Notfall-Beatmungsgeräte, Highflow-Therapiegeräte und Hustenmaschinen.

Das Patienteninterface 90 und das Beatmungsgerät 70 sind bevorzugt über mindestens eine Leitung 80 miteinander verbunden. Die Leitung 80 ist als Gasleitung ausgebildet, die die einzelnen Komponenten des Systems 100 miteinander verbindet. Die Leitung 80 ist bevorzugt flexibel und/oder drehbar ausgebildet. Die Leitung 80 kann beispielsweise als elastisches Rohr und/oder Schlauch und/oder Schlauchsystem ausgebildet sein.

In Fig. 1 sind Patenteninterface 90, Leitung 80 und Beatmungsgerät 70 nicht verbunden dargestellt. Eine gasleitende Verbindung kann hergestellt werden, indem die Leitung 80 beispielsweise über Anschlussstutzen am einen Ende mit dem Patienteninterface 90 und am anderen Ende mit dem Beatmungsgerät 70 verbunden wird (nicht gezeigt).

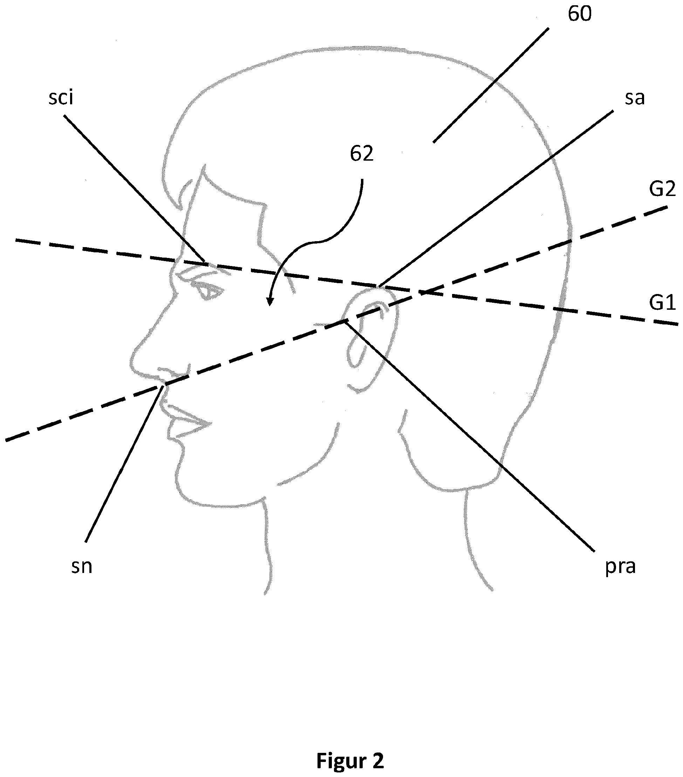

**Fig. 2** zeigt einen menschlichen Kopf 60 in Seitenansicht zur Veranschaulichung von Messpunkten, anhand derer ein Schläfenbereich 62 definiert wird.

Zur Definition der Regionen des menschlichen Kopfes 60 wird hierin auf das Lehrbuch "Anthropologie: Handbuch der vergleichenden Biologie des Menschen" (Fischer Verlag, 4. Auflage, 1988) Bezug genommen. Dort werden die folgenden Meßpunkte am Kopf 60 definiert:
- sa: Höchster Punkt der Ohrmuschel (lat. *Supeaurale*)
- sci: Höchster Punkt des Brauenoberrandes (lat. *Superciliare*)
- pra: Punkt der Ohrbasis (lat. *Praeaurale*)
- sn: Nasenwinkelpunkt (lat. *Subnasale*)

Als Schläfenbereich 62 wird für die vorliegende Patentanmeldung derjenige Bereich definiert, der sich zwischen zwei Geraden G1 und G2 befindet, wobei die Gerade G1 die Meßpunkten sa und sci durchläuft und die Gerade G2 die Messpunkte pra und sn durchläuft (siehe Fig. 1). Der Schläfenbereich 62 kann +/- 3 cm abweichend von dem eingezeichneten Bereich verlaufen.

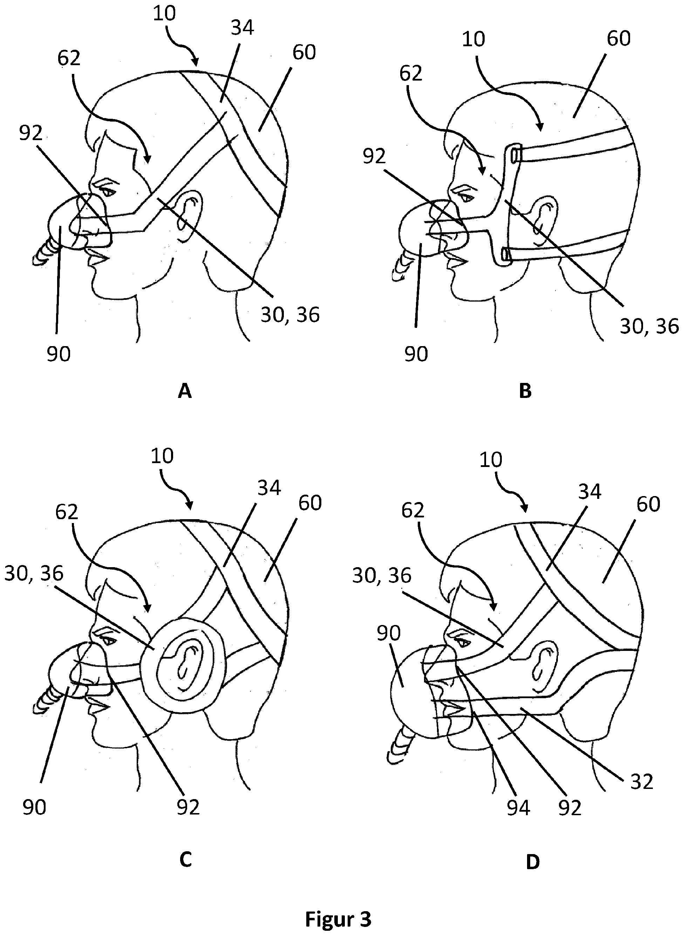

**Fig. 3** zeigt beispielhaft, wie stirnstützenlose Patienteninterfaces 90 über eine erfindungsgemäße Bänderung 10 am Kopf 60 des Anwenders befestigt werden können. Fig. 3 A-C zeigt beispielhaft, wie die Bänderung 10 bei Verwendung eines Patienteninterfaces 90, das als Nasenmaske ausgebildet ist, nach Anlage am Kopf 60 des Anwenders angeordnet sein kann. Fig. 3 D zeigt beispielhaft, wie die Bänderung 10 bei Verwendung eines Patienteninterface 90, das als Full-Face-Maske ausgebildet ist, nach Anlage am Kopf 60 des Anwenders angeordnet sein kann. Die Bänderung 10 kann auch über andere Bereiche des Kopfes 60 verlaufen, der Verlauf der Bänderung 10 ist nicht auf die gezeigten Beispiele beschränkt.

Die erfindungsgemäße Bänderung 10 umfasst mindestens einen Seitenabschnitt 30. Patienteninterfaces 90, die als Nasenmasken ausgebildet sind, enthalten in der Regel jeweils einen Ansatzpunkt 92 je Gesichtshälfte. Über den Ansatzpunkt 92 kann die Bänderung 10 mit dem Patienteninterface 90 verbunden werden. Bevorzugt kann über den Ansatzpunkt 92 der Seitenabschnitt 30 mit dem Patienteninterface 90 verbunden werden. Mehrere Ansatzpunkte 92 sind auch denkbar.

Die Bänderung 10 kann zudem einen Basisabschnitt 34 umfassen (siehe Fig. 3 A, C, D). Der Basisabschnitt 34 kann ausgebildet und eingerichtet sein, eine sichere und komfortable Anlage am Kopf 60 zu gewährleisten. Der Basisabschnitt 34 liegt nach Anlage am Kopf 60 beispielsweise kreisförmig um den Kopf 60 herum und ist zumindest mit dem Seitenabschnitt 30 verbunden.

Patienteninterfaces 90, die als Full-Face-Masken ausgebildet sind, enthalten bevorzugt jeweils zwei Ansatzpunkte 92, 94 je Gesichtshälfte (siehe Fig. 3 D). Es ist jedoch auch möglich, dass Full-Face-Masken nur einen Ansatzpunkt 92 oder auch mehr als zwei Ansatzpunkte 92, 94 aufweisen.

Umfasst das Patienteninterface zwei Ansatzpunkte 92,94, wie in Fig. 3 D gezeigt, kann das Patienteninterface 90 über beide Ansatzpunkte 92, 94 mit der Bänderung 10 verbunden werden. Dadurch können Full-Face-Masken besonders stabil und sicher am Kopf 60 des Anwenders befestigt werden.

Zu diesem Zweck kann die Bänderung 10 neben dem Seitenabschnitt 30 noch einen zweiten Seitenabschnitt 32 umfassen. Bevorzugt kann über den Ansatzpunkt 92 der Seitenabschnitt 30 mit dem Patienteninterface 90 verbunden werden und über den zweiten Ansatzpunkt 94 kann zusätzlich der zweite Seitenabschnitt 32 mit dem Patienteninterface 90 verbunden werden. Der Basisabschnitt 34 ist beispielsweise sowohl mit dem Seitenabschnitt 30 als auch mit dem zweiten Seitenabschnitt 32 verbunden (siehe Fig. 3 D).

Bei Anlage der Atemmaske 90 an den Kopf 60 eines Anwenders verläuft die Bänderung 10 über den Kopf 60 des Anwenders, um die Atemmaske 90 in der gewünschten Position zu halten.

Der Seitenabschnitt 30 verläuft hierbei in der Regel von einem ersten Ansatzpunkt 92 der Atemmaske 90 entlang der Wange und der Schläfe oberhalb des Ohres zum Basisabschnitt 34. Der zweite Seitenabschnitt 32 verläuft in der Regel von einem zweiten Ansatzpunkt 94 der Atemmaske 90 entlang des Unterkiefers und unterhalb des Ohres zum Basisabschnitt 34.

Um eine ausreichende Stabilität zu erreichen, verläuft zumindest ein Bereich der Bänderung 10 bei allen bekannten Bänderungen 10 zumindest bereichsweise oberhalb des Ohres entlang. Somit kreuzt die Bänderung 10 den Schläfenbereich 62 in der Regel mindestens einmal (siehe Figuren 3 A-D).

Der Schläfenbereich 62 ist darüber hinaus derjenige Bereich, über den ein Brillenbügel 64 einer Standardbrille oder Ohrenbrille verläuft (nicht gezeigt). Der Brillenbügel 64 einer Ohrenbrille verläuft normalerweise zwischen dem Augenbereich und dem oberen Ende des Ohrs. Der Brillenbügel 64 verläuft somit längs über den Schläfenbereich 62.

Damit ein Anwender bei Verwendung eines Patienteninterface 90 eine Brille verwenden kann, wird der Bereich der Bänderung 10, der über die Schläfe verläuft, erfindungsgemäß modifiziert. Die Bänderung 10 umfasst somit wenigstens einen Schläfenabschnitt 36. Der Schläfenabschnitt 36 ist derart ausgebildet, dass der Anwender trotz Verwendung eines Patienteninterfaces 90 mit einer Bänderung 10 seine Brille auf- und Absetzen kann (nicht gezeigt).

Der Schläfenabschnitt 36 ist derjenige Abschnitt der Bänderung 10, der nach Anlage am Kopf 60 zumindest bereichsweise über den Schläfenbereich 62 verläuft.

Dieser Schläfenabschnitt 36 ist üblicherweise ein Bereich des Seitenabschnitts 30. Es ist jedoch auch möglich, dass der Schläfenabschnitt 36 ein Abschnitt des Seitenabschnitts 32 und/oder des Basisabschnitts 34 ist. Der Schläfenabschnitt 36 ist derart innerhalb der Bänderung 10 angeordnet, dass der Schläfenabschnitt 36 nach Anlage der Bänderung 10 am Kopf 60 des Anwenders den Schläfenbereich 62 zumindest teilweise überspannt.

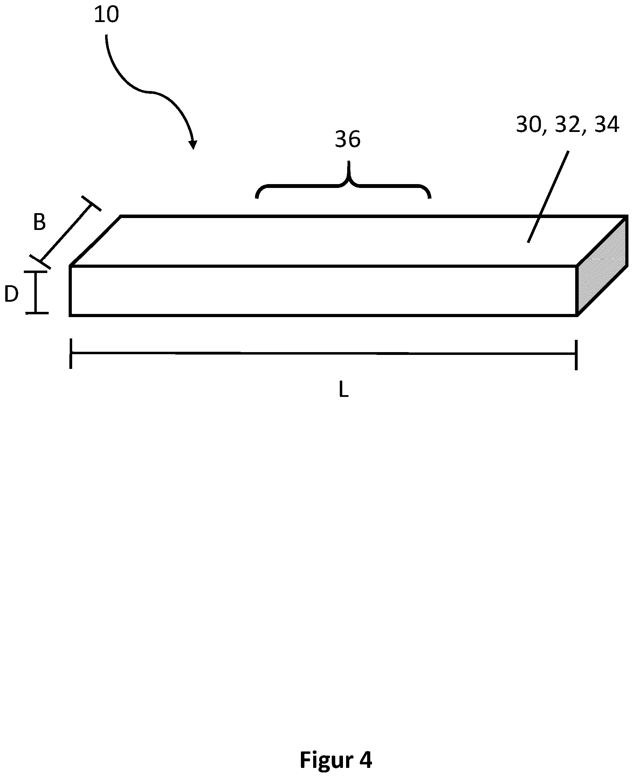

**Fig. 4** zeigt eine perspektivische Draufsicht auf einen Teilbereich eines Seitenabschnitts 30 oder eines zweiten Seitenabschnitts 32 oder eines Basisabschnitts 34 einer erfindungsgemäßen Bänderung 10.

Die Bänderung 10 weist eine Breite B, eine Dicke D und eine Länge L auf. Üblicherweise ist die Breite B immer größer ist als die Dicke D. Die Länge L ist üblicherweise größer als die Breite B.

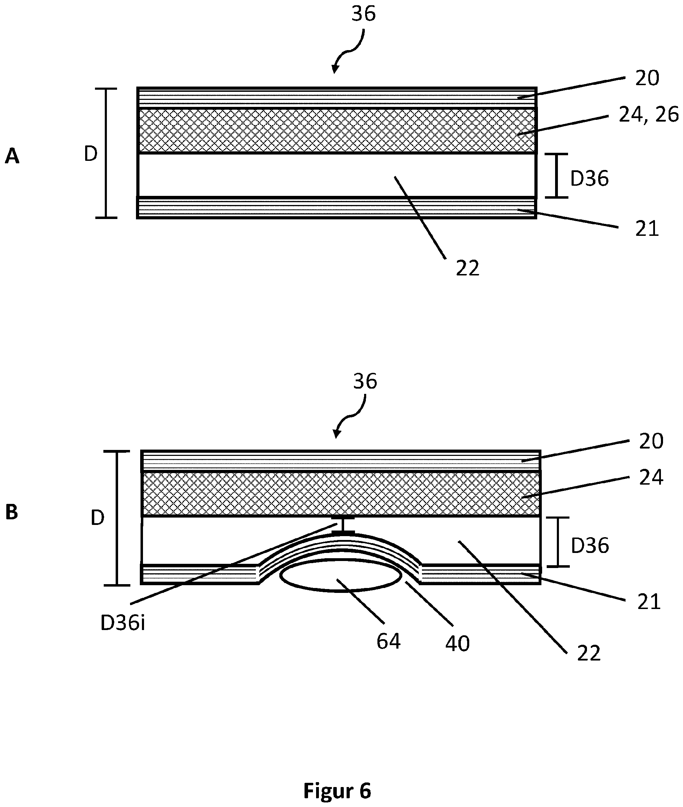

Der gezeigte Teilbereich der Bänderung 10 weist den erfindungsgemäßen Schläfenabschnitt 36 auf, der in den folgenden Figuren 6 bis 9 anhand von Ausführungsbeispielen detaillierter beschrieben wird.

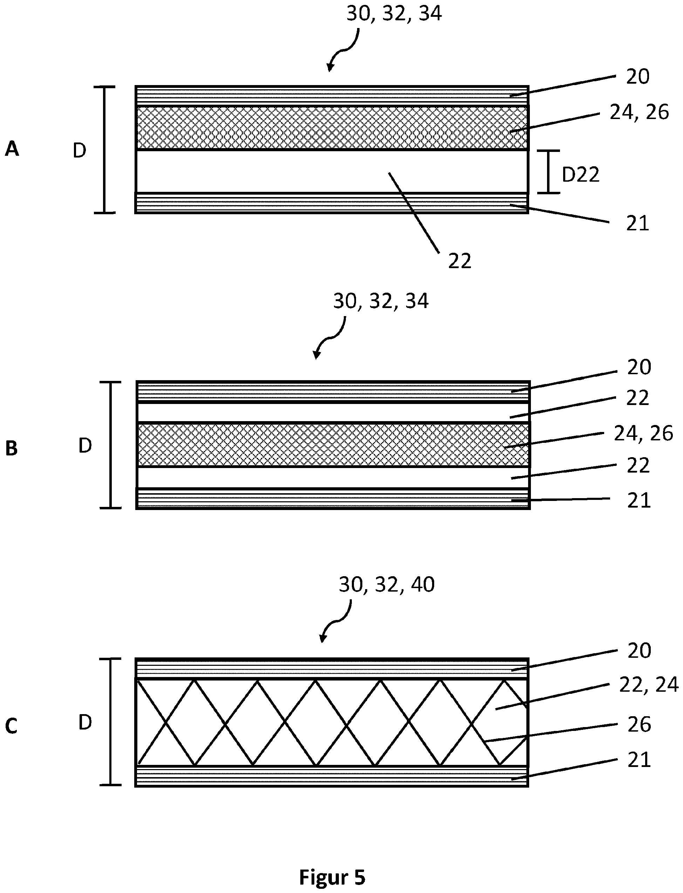

**Fig. 5** zeigt einen Querschnitt durch die Bänderung 10 im Bereich des Seitenabschnitts 30 oder des zweiten Seitenabschnitts 32 oder des Basisabschnitts 34 außerhalb des Schläfenabschnitts 36. Fig. 5 veranschaulicht den generellen Aufbau einer Bänderung 10 für stirnstützenlose Patienteninterfaces 90.

Die Bänderung 10 umfasst zumindest einen Strang 21. Der Strang kann beispielsweise als Stoffstrang 21 ausgebildet sein. In einer einfachen Ausführungsform kann die Bänderung nur einen Stoffstrang 21 umfassen (nicht gezeigt). In einer alternativen Ausführungsform umfasst die Bänderung 10 mindestens zwei Stoffstränge 20, 21. Beispielsweise umfasst die Bänderung 10 einen äußeren Stoffstrang 20 und einen inneren Stoffstrang 21 (Figuren 5 A-C).

Derjenige Stoffstrang, der nach Anlage am Kopf 60 dem Kopf 60 abgewandt ist, wird hierin als äußerer Stoffstrang 20 bezeichnet. Derjenige Stoffstrang, der nach Anlage am Kopf 60 dem Kopf 60 zugewandt ist, wird hierin als innerer Stoffstrang 21 bezeichnet. Der innere Stoffstrang kommt nach Anlage am Kopf 60 zumindest bereichsweise mit der Gesichtshaut des Anwenders in Berührung.

Äußerer Stoffstrang 20 und innerer Stoffstrang 21 können beispielsweise einstückig ausgeführt sein. Äußerer Stoffstrang 20 und innerer Stoffstrang 21 können beispielsweise schlauchförmig ausgebildet sein. Schlauchförmig bedeutet, dass der äußere Stoffstrang 21 und der innere Stoffstrang über die gesamte Länge L entlang der Dicke D miteinander verbunden sind.

Es ist auch denkbar, dass äußerer Stoffstrang 20 und innerer Stoffstrang 21 zweistückig ausgeführt sind. In manchen Ausführungsformen können der äußere Stoffstrang 20 und der innere Stoffstrang 21 zumindest bereichsweise miteinander verbunden sein. Beispielsweise können der äußere Stoffstrang 20 und der innere Stoffstrang 21 zumindest bereichsweise miteinander verklebt, verschweißt, vernäht oder ähnliches sein.

Äußerer Stoffstrang 20 und innerer Stoffstrang 21 können aus demselben Material gefertigt sein. Äußerer Stoffstrang 20 und innerer Stoffstrang 21 können auch aus unterschiedlichen Materialien gefertigt sein.

Der Stoffstrang 20, 21 ist beispielsweise aus einem Textil wie Baumwolle, Seide und/oder aus synthetischen Fasern wie beispielsweise aus Polyester, Polyurethan, Neopren, Spandex und/oder Nylon gefertigt. Mischgewebe sind auch denkbar. Denkbar sind auch andere Stoffe, die in Bezug auf Biokompatibilität, Komfort, Stabilität, Elastizität und Kosten geeignet erscheinen.

In manchen Ausführungsformen sind der äußere Stoffstrang 20 und der innerer Stoffstrang 21 aus dem gleichen Material gefertigt, beispielsweise aus Nylon mit Polyester und/oder aus Nylon und Spandex.

Zusätzlich zum mindestens einen Stoffstrang 20, 21 umfasst die Bänderung 10 bevorzugt mindestens eine weitere Schicht. Die weitere Schicht und/oder Schichten können beispielsweise abpolsternde, verstärkende und/oder andere vorteilhafte Eigenschaften aufweisen. Äußerer Stoffstrang 20 und innerer Stoffstrang 21 können in manchen Ausführungsformen mit mindestens einer weiteren Schicht als Abstandsgewirk ausgebildet sein.

Bänderungen 10 können vorzugsweise zumindest bereichsweise zumindest eine Polsterschicht 22 aufweisen, um den Tragekomfort zu erhöhen.

Die Polsterschicht 22 ist beispielsweise aus Schaumstoff, Watte, Gewebe, Baumwolle, Wolle, Gummi, Neopren, Gel und/oder anderen elastischen und/oder viskoelastischen Materialien gefertigt.

Die Polsterschicht 22 weist eine Dicke D22 auf. Die Dicke D22 kann beispielsweise konstant sein. Die Dicke D22 kann in alternativen Ausführungsbeispielen in unterschiedlichen Abschnitten der Bänderung 10 auch unterschiedliche Dicken aufweisen.

Die Dicke D22 der Polsterschicht 22 liegt beispielsweise in einem Bereich von 0,5 mm bis 6 mm. Bevorzugt liegt die Dicke D22 der Polsterschicht 22 in einem Bereich von 1,5 mm bis 3 mm.

In manchen Ausführungsformen kann die Polsterschicht 22 auf dem äußeren Stoffstrang 20 und/oder dem inneren Stoffstrang 21 aufgebracht sein. Die Polsterschicht 22 kann sodann bei Anlage am Kopf 60 zumindest bereichsweise auf der Haut des Anwenders aufliegen (nicht gezeigt). Die Polsterschicht 22 kann in alternativen Ausführungsformen auch in den Stoffstrang 20,21 integriert sein (nicht gezeigt). In manchen Ausführungsformen ist die Polsterschicht 22 zwischen dem äußeren Stoffstrang 20 und dem inneren Stoffstrang 21 angeordnet und bildet mindestens eine Zwischenschicht (Figuren 5 A-C).

Stirnstützenlose Bänderungen 10 weisen vorzugsweise zumindest bereichsweise zumindest eine Verstärkungsschicht 24 auf (siehe Fig. 5 A - C). Die Verstärkungsschicht 24 sollte eine gewisse Steifigkeit bei gleichzeitiger Flexibilität aufweisen, um der Bänderung 10 eine ausreichende Stabilität zu verleihen und trotzdem eine Anlage am Kopf 60 zu ermöglichen.

Die Verstärkungsschicht 24 kann aus verstärkenden Elementen 26 bestehen oder diese umfassen. Die verstärkenden Elemente 26 können die Verstärkungsschicht 24 darstellen. Die verstärkenden Elemente 26 können auch in ein Grundmaterial der Verstärkungsschicht 24 integriert sein.

Die Verstärkungsschicht 24 und/oder die verstärkenden Elemente 26 können auf dem Stoffstrang 20,21 aufgebracht sein (nicht gezeigt). Die Verstärkungsschicht 24 und/oder die verstärkenden Elemente 26 können in den Stoffstrang 20,21 integriert sein (nicht gezeigt). In einer alternativen Ausführungsform sind die Verstärkungsschicht 24 und/oder die verstärkenden Elemente 26 zwischen dem äußeren Stoffstrang 20 und dem inneren Stoffstrang 21 angeordnet und bilden eine Zwischenschicht (Figuren 5 A-C).

Die Verstärkungsschicht 24 und/oder die verstärkenden Elemente 26 sind beispielsweise aus Kunststoff, Metall, Silikon, Schaumstoff und/oder einem Materialverbund ausgebildet.

Die Verstärkungsschicht 24 und/oder die verstärkenden Elemente 26 können in Form von Hartplastik, Draht, anorganischen und/oder organischen Verstärkungsfasern, Kurz- und/oder Langfasern, Stäben, Streifen, Gewebe, Geflecht, Gitter und/oder Strukturmatten ausgebildet sein.

In manchen Ausführungsformen kann die Verstärkungsschicht 24 als verformbares Element ausgebildet sein, das individuell an die Kopfform angepasst werden kann. Beispielsweise kann die Verstärkungsschicht 24 aus mindestens einem Draht aus einer Knetlegierung ausgebildet sein, beispielsweise aus einer Aluminium-Knetlegierung. Andere Materialien mit verformbaren Eigenschaften sind auch denkbar, beispielsweise Kunststoffe wie Thermoplaste oder ähnliche.

Die Verstärkungsschicht 24 und/oder die verstärkenden Elemente 26 sind bevorzugt aus einem Hartkunststoff gefertigt. Die Verstärkungsschicht 24 und/oder die verstärkenden Elemente 26 sind beispielsweise aus Polypropylen gefertigt.

In einer alternativen Ausführungsform sind die Verstärkungsschicht 24 und/oder die verstärkenden Elemente 26 aus einem Materialverbund ausgebildet, vorzugsweise aus einem Hartkunststoff und Silikon und/oder aus einem Hartkunststoff und einem thermoplastischen Elastomer (TPE).

Die Verstärkungsschicht 24 und/oder die verstärkenden Elemente 26 sind nicht auf diese Ausführungsformen beschränkt. Jegliche Materialien, die verstärkende und/oder versteifende Eigenschaften aufweisen sind denkbar.

Die weiteren Schichten wie beispielsweise die Polsterschicht 22 und/oder die Verstärkungsschicht 24 können von außen auf den Stoffstrang 20, 21 aufgebracht werden und/oder in den Stoffstrang 20, 21 integriert werden und/oder zwischen zwei Stoffsträngen 20, 21 angeordnet sein. Beispielsweise kann zwischen dem inneren Stoffstrang 21 und dem äußeren Stoffstrang 20 mindestens eine Zwischenschicht ausgebildet sein (siehe Fig. 5 A-C).

Die Verbindung des inneren Stoffstrangs 21 mit dem äußeren Stoffstrangs 20 und/oder den Zwischenschichten kann lösbar und/oder unlösbar erfolgen.

Die Verbindung des inneren Stoffstrangs 21 mit dem äußeren Stoffstrangs 20 und/oder den Zwischenschichten kann beispielsweise per Kleben, Schweißen, Kletten, Nähen, Kaschieren und/oder Flammkaschieren erfolgen. Es ist auch denkbar, dass in der Polsterschicht 22 eine Tasche ist, in die die Verstärkungsschicht 24 eingebracht wird. Die Polsterschicht 22 kann auch einen Umgriff um die Verstärkungsschicht 24 aufweisen und so auf die Verstärkungsschicht 24 aufgeklickt werden.

Die Verbindung zwischen dem Stoffstrang 20, 21 und der Polsterschicht kann beispielsweise per Kaschierung erfolgen. Dabei wird die Verbindung mittels nassem oder trockenem Klebstoff hergestellt. Es ist aber auch möglich, die Verbindung ohne Klebstoff nur durch Wärme und Druck herzustellen, beispielsweise durch Flammkaschieren.

Fig. 5 A-C zeigen beispielhafte Ausführungsformen, bei denen die Bänderung 10 derart ausgebildet ist, dass zwischen dem äußeren Stoffstrang 20 und dem inneren Stoffstrang 21 mindestens eine Zwischenschicht angeordnet ist.

In der Ausführungsform nach Fig. 5 A ist benachbart zum äußeren Stoffstrang 20 eine Verstärkungsschicht 24 mit verstärkenden Elementen 26 angeordnet. Benachbart zum inneren Stoffstrang 21 ist eine Polsterschicht 22 angeordnet. Weitere Schichten sind in dieser Ausführungsform nicht vorgesehen. Somit sind Verstärkungsschicht 24 und Polsterschicht 22 nebeneinander angeordnet und bilden gemeinsam die Zwischenschicht, die zwischen dem äußeren Stoffstrang 20 und dem inneren Stoffstrang 21 angeordnet ist.

In der Ausführungsform nach Fig. 5 B ist benachbart zum äußeren Stoffstrang 20 eine erste Polsterschicht 22 angeordnet. Benachbart zum inneren Stoffstrang 21 ist eine zweite Polsterschicht 22 angeordnet. Zwischen den beiden Polsterschichten 22 ist die Verstärkungsschicht 24 mit verstärkenden Elementen 26 angeordnet. Weitere Schichten sind in dieser Ausführungsform nicht vorgesehen. Somit ist die Verstärkungsschicht 24 zwischen zwei Polsterschichten 22 angeordnet. Die Verstärkungsschicht 24 und die zwei Polsterschichten 22 bilden gemeinsam die Zwischenschicht, die zwischen dem äußeren Stoffstrang 20 und dem inneren Stoffstrang 21 angeordnet ist.

In der Ausführungsform nach Fig. 5 C ist zwischen dem äußeren Stoffstrang 20 und dem inneren Stoffstrang 21 eine Polsterschicht 22 angeordnet, in die die verstärkenden Elemente 26 integriert sind. Somit ist die Polsterschicht 22 auch als Verstärkungsschicht 24 ausgebildet. Zwischen dem äußeren Stoffstrang 20 und dem inneren Stoffstrang 21 ist demnach eine Zwischenschicht ausgebildet, die gleichzeitig verstärkende und abpolsternde Eigenschaften aufweist.

Auf Grundlage aller dargestellten Ausführungsbeispiele der Fig. 5 kann der speziell geformte Schläfenabschnitt 36 ausgebildet werden.

Die hier offenbarte Bänderung 10 schließt ausdrücklich auch Bänderungen 10 mit ein, deren genereller Aufbau nicht figürlich in Fig. 5 dargestellt sind, wie beispielsweise Bänderungen 10, bei denen die Polsterschicht 22 und/oder die Verstärkungsschicht 24 auf dem äußeren Stoffstrang 20 und/oder dem inneren Stoffstrang 21 aufgebracht sind und/oder in den Stoffstrang 20,21 integriert sind.

**Fig. 6 bis 10** zeigen verschiedene Ausführungsbeispiele des Schläfenabschnitts 36 der Bänderung 10 im Querschnitt. Die Bänderung 10 weist erfindungsgemäß zumindest bereichsweise einen Brillentunnel 40 auf, der eingerichtet und ausgebildet ist zumindest einen Brillenbügel 64 zumindest teilweise aufzunehmen.

Der Brillentunnel 40 kann, wie anhand der nachfolgenden Ausführungsbeispiele erläutert wird, reversibel und/oder irreversibel ausgebildet sein.

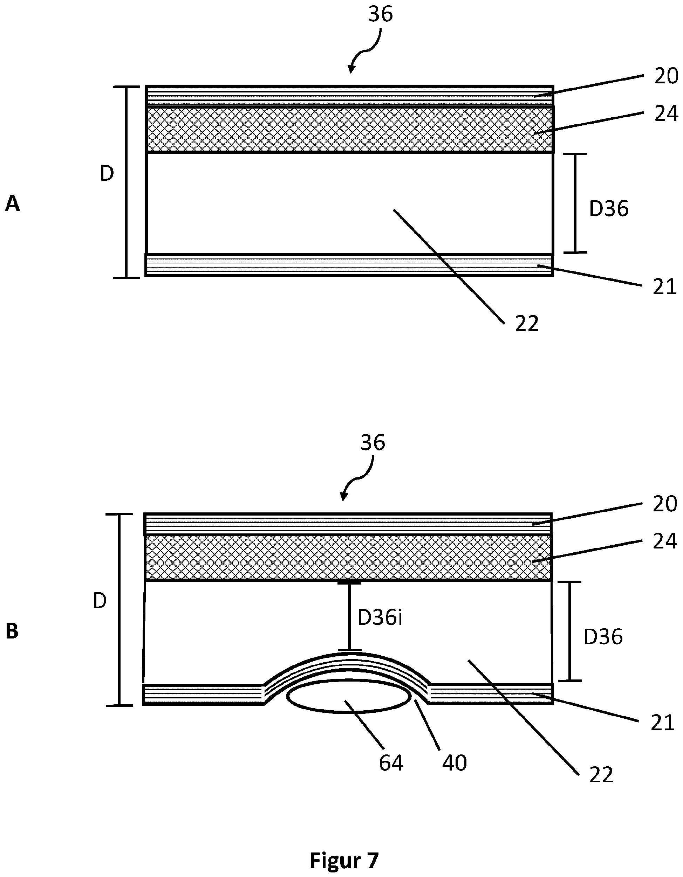

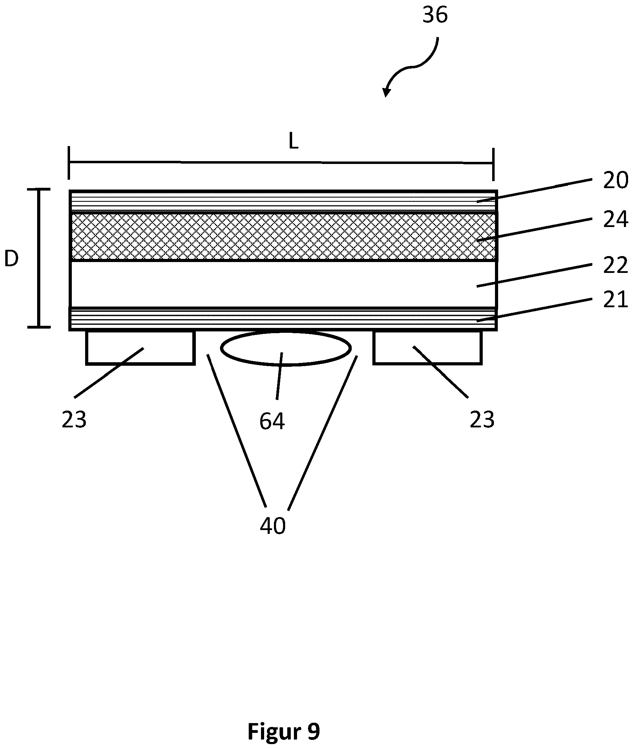

Ein reversibel ausgebildeter Brillentunnel 40 entsteht hierbei durch die Einwirkung des Brillenbügels 64 (siehe Fig. 6, 7) und/oder durch einen lösbaren Zusatz zur Bänderung 10 (siehe Fig. 9).

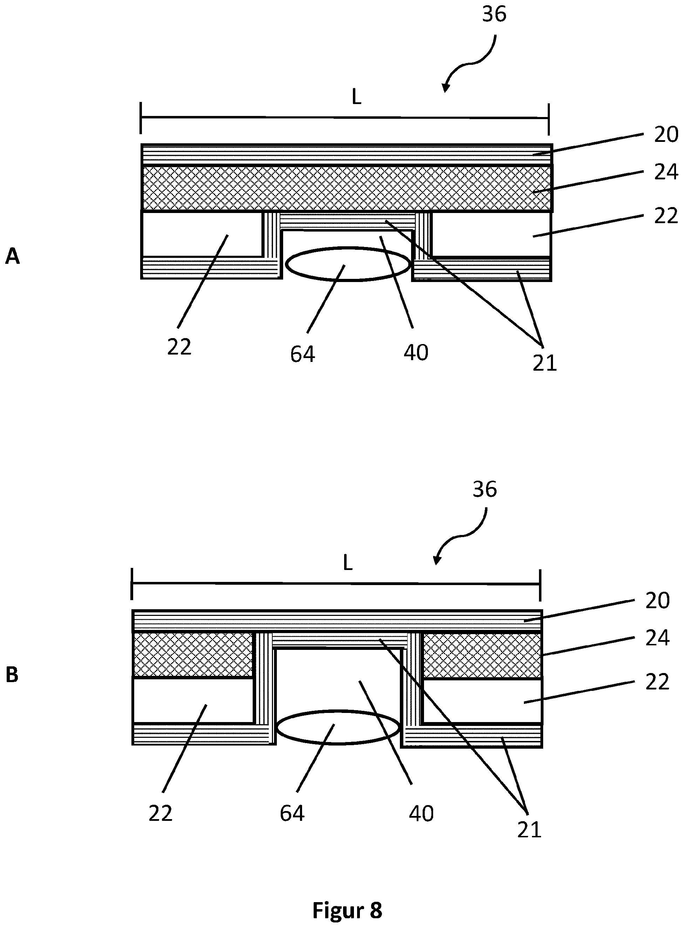

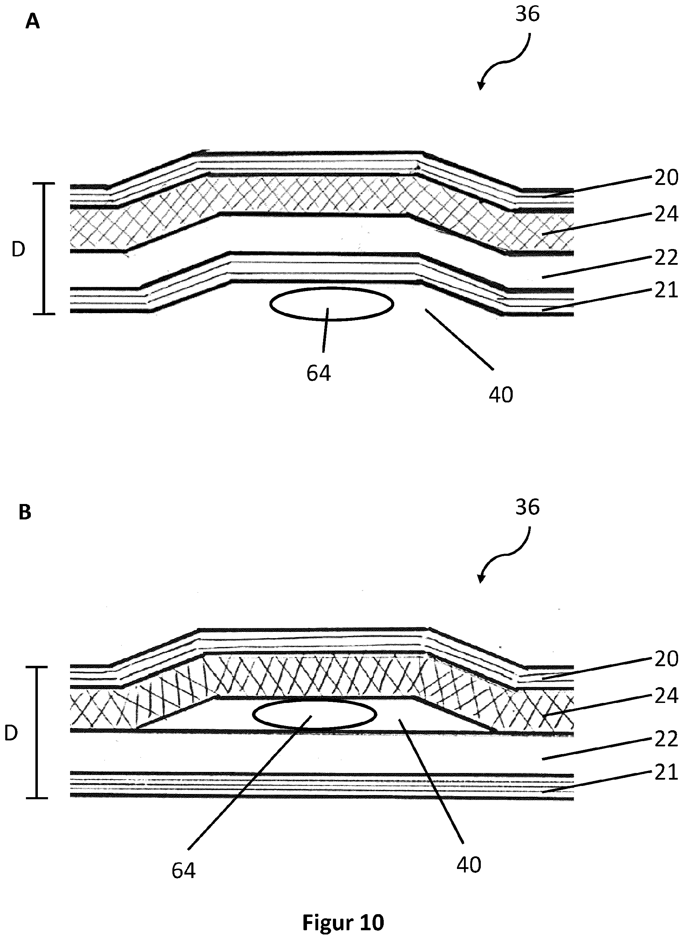

Ein irreversibel ausgebildeter Brillentunnel 40 entsteht hingegen durch einen unlösbaren Zusatz zur Bänderung 10 (siehe Fig. 9) und/oder durch eine zumindest teilweise Aussparung im inneren Stoffstrang 21 und/oder in der Verstärkungsschicht 24 und/oder in der Polsterschicht 22 und/oder im äußeren Stoffstrang 20 (siehe Fig. 8) und/oder durch eine zumindest abschnittsweise und/oder zumindest teilweise Vorformung der Bänderung 10 (siehe Fig. 10).

**Fig. 6** zeigt eine Ausführungsform für den Schläfenabschnitt 36 im Querschnitt. Aus Fig. 6 wird ersichtlich, dass der generelle Aufbau dem in Fig. 5 A dargestellten Aufbau der Bänderung 10 entsprechen kann. Der generelle Aufbau kann in anderen Ausführungsformen auch dem in Fig. 5 B und/oder C dargestellten Aufbau entsprechen oder derart sein, dass die Polsterschicht 22 und/oder die Verstärkungsschicht 24 auf den Stoffstrang 20, 21 aufgebracht und/oder integriert ist (nicht gezeigt).

Aus Figur 6 wird ersichtlich, dass die Polsterschicht 22 im Schläfenabschnitt 36 eine Dicke D36 aufweist. Die Dicke D36 kann der Dicke D22 der Polsterschicht 22 der übrigen Abschnitte der Bänderung 10 entsprechen. Die Dicke D22/D36 der Polsterschicht 22 kann demnach konstant sein.

In dieser beispielhaften Ausführungsform weist die Polsterschicht 22 und/oder die Verstärkungsschicht 24 und/oder der Stoffstrang 20, 21 zumindest bereichsweise eine materialbedingte Schwachstelle auf.

In einer beispielhaften Ausführungsform kann die Polsterschicht 22 im Schläfenabschnitt 36 weicher ausgebildet sein als in den anderen Abschnitten der Bänderung 10 (Fig. 6 A). "Weicher" bedeutet in diesem Zusammenhang, dass für ein Zusammendrücken der Polsterschicht 22 im Schläfenabschnitt 36 eine geringere Kraft aufgewendet werden muss, als für ein Zusammendrücken der Polsterschicht 22 in anderen Abschnitten der Bänderung 10.

Beispielsweise ist die Kraft, die benötigt wird, die Polsterschicht 22 um 25% auf die Dicke D36i zusammenzudrücken (siehe Fig. 6 B) im Schläfenabschnitt 36 um mindestens 10 % kleiner, bevorzugt um mindestens 20 % kleiner, besonders bevorzugt um mindestens 30 % kleiner als in den anderen Abschnitten der Bänderung. Der Härtegrad des Polstermaterials ist im Schläfenabschnitt 36 demnach geringer als in den anderen Abschnitten der Bänderung 10.

Der Härtegrad des Polstermaterials ist im Schläfenabschnitt 36 derart gewählt, dass ein Brillenbügel 64 (nicht gezeigt), der unter dem Schläfenabschnitt 36 verläuft, das Polstermaterial derart leicht verdrängt, dass es zu keinem unangenehmen Druck des Brillenbügels 64 auf den Kopf 60 des Anwenders kommt.

Wenn ein Brillenbügel 64, der unter dem Schläfenabschnitt 36 verläuft, die Polsterschicht 22 auf die Dicke D36i verdrängt, entsteht ein Brillentunnel 40. Der Brillentunnel 40 entsteht durch den Druck, den ein Brillenbügel 64 auf die Polsterschicht 22 ausübt.

Der Brillentunnel 40 bildet sich in dieser Ausführungsform reversibel, sobald sich ein Brillenbügel 64 unter dem Schläfenabschnitt 36 befindet. Wird der Brillenbügel 64 entfernt, weitet sich die Polsterschicht 22 wieder auf ihre ursprüngliche Dicke D36 aus und der Brillentunnel 40 verschwindet.

Die Polsterschicht 22 der Bänderung 10 kann aus einem Material oder aus mindestens zwei verschiedenen Materialien ausgebildet sein.

In manchen Ausführungsformen ist es vorgesehen, dass die Polsterschicht 22 im Schläfenabschnitt 36 aus einem anderen Material gefertigt ist als die Polsterschicht 22 in den anderen Abschnitten der Bänderung 10, um im Schläfenabschnitt 36 eine weichere Polsterung als im Rest der Bänderung 10 zu erreichen.

In anderen Ausführungsformen ist das Material der Polsterschicht 22 im Schläfenabschnitt 36 dasselbe wie in den anderen Abschnitten der Bänderung 10, weist jedoch zumindest bereichsweise andere Materialeigenschaften auf. Beispielsweise kann die Polsterschicht 22 im Schläfenabschnitt 36 eine differierende Materialdichte und/oder einen anderen chemischen Aufbau aufweisen. Der chemische Aufbau kann beispielsweise bezüglich der Molekülkettenlänge, Anzahl der Vernetzungen innerhalb der Molekülketten, Aufbau der Molekülketten und ähnliches differieren, so dass das Material der Polsterschicht 22 im Schläfenabschnitt weicher ausgebildet ist als in den anderen Abschnitten der Bänderung 10.

In Ausführungsformen, in denen die Polsterschicht 22 aus Schaumstoff ausgebildet ist, kann der Schaumstoff beispielsweise derart unterschiedliche Porenanzahlen und/oder Porengrößen aufweisen, dass die Polsterschicht 22 im Schläfenabschnitt 36 weicher ausgebildet ist als in den anderen Abschnitten der Bänderung 10.

Eine weichere Polsterschicht 22 im Schläfenabschnitt 36 ist auch für den generellen Aufbau der Ausführungsformen nach Fig. 5 B und/oder C möglich.

In Bezug auf den Aufbau nach Fig. 5C kann das bedeuten, dass die eine Zwischenschicht, die gleichzeitig verstärkende und abpolsternde Eigenschaften aufweist, im Schläfenabschnitt 36 eine Schwachstelle aufweist. Beispielsweise kann die Zwischenschicht im Schläfenabschnitt 36 weniger und/oder keine verstärkenden Eigenschaften aufweisen (nicht gezeigt).

**Fig. 7** zeigt eine alternative Ausführungsform des Schläfenabschnitts 36 im Querschnitt. Aus Fig. 7 wird ersichtlich, dass der generelle Aufbau dem in Fig. 5 A dargestellten Aufbau der Bänderung 10 entsprechen kann. Der generelle Aufbau kann in anderen Ausführungsformen auch dem in Fig. 5 B und/oder C dargestellten Aufbau entsprechen oder derart sein, dass die Polsterschicht 22 und/oder die Verstärkungsschicht 24 auf den Stoffstrang 20, 21 aufgebracht und/oder integriert ist (nicht gezeigt).

Aus Fig. 7 A wird jedoch ersichtlich, dass in dieser Ausführungsform die Polsterschicht 22 im Bereich des Schläfenabschnitts 36 eine größere Dicke D36 aufweist als in den Abschnitten der Bänderung 10, die sich nicht im Schläfenabschnitt 36 befinden (D22, vgl. Fig. 5A).

Die Dicke D36 der Polsterschicht 22 kann im Schläfenabschnitt 36 kann beispielsweise mindestens 1 mm dicker sein als die Dicke D22 in den anderen Abschnitten der Bänderung 10. Bevorzugt ist die Dicke D36 der Polsterschicht 22 im Schläfenabschnitt 36 um mindestens 3 mm dicker als die Dicke D22 in den anderen Abschnitten der Bänderung 10. Beispielsweise ist die Dicke D22 der Polsterschicht 22 der Bänderung 10 generell 1,5 bis 3mm dick und steigt im Schläfenabschnitt 36 auf eine Dicke D36 von 4,5 bis 6 mm an.

Die Dicke D36 der Polsterschicht 22 kann im Schläfenabschnitt 36 sprunghaft ansteigen. Die Dicke D36 der Polsterschicht 22 kann in einer alternativen Ausführungsform auch derart sukzessive ansteigen, dass die Polsterschicht 22 im Schläfenabschnitt die Dicke D36 aufweist und dort konstant ist (nicht gezeigt).

Die Dicke D36 ist derart dick gewählt, dass ein Brillenbügel 64 (nicht gezeigt), der unter dem Schläfenabschnitt 36 verläuft, die Polsterschicht 22 derart verdrängt, dass die Polsterschicht 22 in dem Bereich eine Dicke D36i aufweist (siehe Fig. 7 B). Die Dicke D36i ist kleiner als die Dicke D36.

Wenn ein Brillenbügel 64, der unter dem Schläfenabschnitt 36 verläuft, die Polsterschicht 22 auf die Dicke D36i verdrängt, entsteht der Brillentunnel 40.

Der Brillentunnel 40 entsteht durch den Druck, den ein Brillenbügel 64 auf die Polsterschicht 22 ausübt.

Der Brillentunnel 40 wird in dieser Ausführungsform reversibel gebildet. Wenn der Brillenbügel 64 aus dem Schläfenabschnitt 36 entfernt wird, kann sich die Polsterschicht 22 in dem Bereich wieder auf die ursprüngliche Dicke D36 ausdehnen.

Eine dickere Polsterschicht 22 im Schläfenabschnitt 36 ist auch für andere Bänderungen 10, beispielsweise entsprechend der Ausführungsformen nach Fig. 5 B und/oder C möglich.

**Fig. 8** zeigt eine alternative Ausführungsform des Schläfenabschnitts 36 im Querschnitt. Aus Fig. 8 wird ersichtlich, dass der generelle Aufbau dem in Fig. 5 A dargestellten Aufbau der Bänderung 10 entsprechen kann. Der generelle Aufbau kann in anderen Ausführungsformen auch dem in Fig. 5 B und/oder C dargestellten Aufbau entsprechen oder derart sein, dass die Polsterschicht 22 und/oder die Verstärkungsschicht 24 auf den Stoffstrang 20, 21 aufgebracht und/oder integriert ist (nicht gezeigt).

Aus Fig. 8 wird ersichtlich, dass die Bänderung 10 in dieser Ausführungsform mindestens eine Aussparung aufweist. Die Aussparung kann zumindest teilweise in der Polsterschicht 22 und/oder in der Verstärkungsschicht 24 und/oder in dem Stoffstrang 20, 21 ausgebildet sein. Somit kann in manchen Ausführungsformen die Polsterschicht 22 und/oder der Verstärkungsschicht 24 und/oder dem Stoffstrang 20, 21 im Schläfenabschnitt 36 dünner ausgebildet sein, als in den anderen Abschnitten der Bänderung 10 (nicht gezeigt).

In einer alternativen Ausführungsform kann die Aussparung die gesamte Polsterschicht 22 und/oder die Verstärkungsschicht 24 und/oder den Stoffstranges 20, 21 betreffen. Beispielsweise kann die Polsterschicht 22 und/oder die Verstärkungsschicht 24 im Bereich des Schläfenabschnitts 36 eine Aussparung aufweisen (siehe Fig. 8 A und B). Das bedeutet, dass sich die Polsterschicht 22 und/oder die Verstärkungsschicht 24 nicht über die gesamte Länge L des Schläfenabschnitts 36 erstrecken.

Die Aussparung kann insgesamt 20 % bis 100 % der Länge L des Schläfenabschnitts 36 umfassen, bevorzugt 50 % bis 100 %. Beispielsweise umfasst die Aussparung 80 % der Länge L des Schläfenabschnitts 36.

In einer Ausführungsform entsteht der Brillentunnel 40 durch die Aussparung in der Polsterschicht 22 (Fig. 8 A). Alternativ oder zusätzlich kann auch die Verstärkungsschicht 24 eine entsprechende Aussparung aufweisen (Fig. 8 B). Der Brillentunnel 40 ist in diesem Ausführungsbeispiel vorgeformt und irreversibel.

Der Brillentunnel 40 kann durch die Aussparung zwischen 3 mm und 30 mm hoch ausgebildet sein. Bevorzugt ist der Brillentunnel 40 zwischen 5 mm und 20 mm hoch ausgebildet.

Im Bereich des Brillentunnels 40 kann der innere Stoffstrang 21 direkt mit der Verstärkungsschicht 24 verbunden sein. Der Brillentunnel 40 kann sodann von dem inneren Stoffstrang 21 begrenzt sein (siehe Fig. 8 A). Der innere Stoffstrang 21 kann auch direkt mit dem äußeren Stoffstrang 20 verbunden sein. Der Brillentunnel 40 kann sodann von dem inneren Stoffstrang 21 begrenzt sein (siehe Fig. 8 B).

Es ist auch denkbar, dass auch der innere Stoffstrang 21 im Bereich des Brillentunnels 40 eine der Polsterschicht 22 und/oder der Verstärkungsschicht 24 entsprechende Aussparung aufweist (nicht gezeigt). Sodann würde der Schläfenabschnitt 36 im Bereich des Brillentunnels 40 ausschließlich die Verstärkungsschicht 24 und/oder den äußeren Stoffstrang 20 umfassen.

Der Brillentunnel 40 erstreckt sich in dieser Ausführungsform beispielsweise über 20 % bis 100 % der Länge L des Schläfenabschnitts 36, bevorzugt über 50 % bis 100 %. Beispielsweise erstreckt sich der Brillentunnel 40 über 80 % der Länge L des Schläfenabschnitts 36.

Der Brillentunnel 40 ist ausgebildet und eingerichtet, dass der Anwender einen Brillenbügel 64 durch den Brillentunnel 40 hindurch und/oder aus dem Brillentunnel herausführen kann, so dass eine zeitgleiche Verwendung eines Patienteninterface 90 mit Bänderung 10 und einer Brille ermöglicht wird.

**Fig. 9** zeigt eine alternative Ausführungsform des Schläfenabschnitts 36 im Querschnitt. Aus Fig. 9 wird ersichtlich, dass der generelle Aufbau dem in Fig. 5 A dargestellten Aufbau der Bänderung 10 entsprechen kann. Der generelle Aufbau kann in anderen Ausführungsformen auch dem in Fig. 5 B und/oder C dargestellten Aufbau entsprechen oder derart sein, dass die Polsterschicht 22 und/oder die Verstärkungsschicht 24 auf den Stoffstrang 20, 21 aufgebracht und/oder integriert ist (nicht gezeigt).

In diesem Ausführungsbeispiel ist vorgesehen, dass im Schläfenabschnitt 36 ein oder mehrere Distanzpolster 23 angeordnet sind.

In einer einfachen Ausführungsform umfasst der Schläfenabschnitt 36 ein Distanzpolster 23, das derart im Schläfenabschnitt 36 angeordnet ist, dass nach Anlage der Bänderung am Kopf 60 eine Lücke zwischen Bänderung 10 und Kopf 60 entsteht, durch die ein Brillenbügel 64 hindurchgeführt werden kann (nicht gezeigt).

In einer bevorzugten Ausführungsform umfasst der Schläfenabschnitt 36 zwei Distanzpolster 23. Die beispielsweise zwei Distanzpolter 23 können derart beabstandet voneinander angeordnet sein, dass der Brillentunnel 40 entsteht (siehe Fig. 9).

Durch die Distanzpolster 23 kann der Brillentunnel 40 vorgeformt werden.

Die Distanzpolster 23 können beispielsweise aus dem gleichen Material gefertigt sein wie die Polsterschicht 22. Die Distanzpolster 23 können auch aus einem anderen Material als die Polsterschicht 22 ausgebildet sein, das in Bezug auf Biokompatibilität und Komfort geeignet erscheint.

Die Distanzpolster 23 können beispielsweise die gleiche Dicke aufweisen wie die Polsterschicht 22. Die Distanzpolster 23 können auch dicker oder dünner als die Polsterschicht 22 ausgebildet sein. Beispielsweise kann der Brillentunnel 40 durch die Distanzpolster 23 zwischen 3 mm und 30 mm hoch ausgebildet sein. Bevorzugt ist der Brillentunnel 40 zwischen 5 mm und 20 mm hoch ausgebildet.

Die Distanzpolster 23 können an dem inneren Stoffstrang 21 angeformt sein. Die Distanzpolster 23 können in alternativen Ausführungsformen jedoch auch innerhalb des Stoffstranges 20,21 angeordnet sein, beispielsweise zwischen dem innerem Stoffstrang 21 und der Polsterschicht 22 (nicht gezeigt).

Die Distanzpolster 23 sind mit der Bänderung 10 zumindest bereichsweise verbunden. Die Distanzpolster sind bevorzugt zumindest bereichsweise mit dem Schläfenabschnitt 36 verbunden.

Die Distanzpolster 23 können in manchen Ausführungsformen nur an einer Seite der Bänderung 10 angeordnet sein. Beispielsweise sind die Distanzpolster 23 derart angeordnet, dass sie sich nach Anlage am Kopf 60 des Anwenders (nicht gezeigt) zwischen Bänderung 10 und Kopf 60 befinden. Die Distanzpolster 23 können in diesem Falle beispielsweise auf dem inneren Stoffstrang 21 angeordnet sein (siehe Fig. 9).

In einem alternativen Ausführungsbeispiel ist es vorgesehen, dass das mindestens eine Distanzpolster 23 im Schläfenabschnitt 36 ringförmig um die Bänderung 10 herum angeordnet ist (nicht gezeigt).

Die Distanzpolster 23 können fest und irreversibel mit dem Schläfenabschnitts 36 verbunden sein. Beispielsweise können die Distanzpolster 23 an Bereiche des Schläfenabschnitts 36 geklebt, genäht, eingenäht, geschweißt, geheftet oder ähnliches sein.

In einer besonders vorteilhaften Ausführungsform können die Distanzpolster 23 auch reversibel mit dem Schläfenabschnitt 36 verbunden sein. Beispielsweise ist denkbar, dass die Distanzpolster 23 über einen Klettverschluss oder eine andere reversible Verbindung mit dem Schläfenabschnitt 36 verbunden werden. Eine weitere Möglichkeit bieten ringförmige Distanzpolster 23. Diese können vom Anwender bei Bedarf auf die Bänderung 10 geschoben und im Schläfenabschnitt 36 platziert werden. Eine reversible Verbindung bietet dem Anwender den Vorteil einer individuellen Anpassung an seinen Bedarf. Auf diese Weise kann der Anwender den Brillentunnel 40 an die verwendete Brille anpassen. Zudem kann der Anwender den Brillentunnel 40 nur dann bereitstellen, wenn eine Brille verwendet wird.

Die Distanzpolster 23 sind insgesamt beispielsweise über 20 % bis 80 % der Länge L des Schläfenabschnitts 36 angeordnet, bevorzugt über 40 % bis 70 %. Beispielsweise sind die Distanzpolster 23 insgesamt über 50 % der Länge L des Schläfenabschnitts 36 angeordnet.

Der Brillentunnel 40, der durch die Distanzpolster 23 gebildet wird, erstreckt sich in dieser Ausführungsform beispielsweise über 80 % bis 20 % der Länge L des Schläfenabschnitts 36, bevorzugt über 60 % bis 30 %. Beispielsweise erstreckt sich der Brillentunnel 40 über 50 % der Länge L des Schläfenabschnitts 36.

**Fig. 10** zeigt eine alternative Ausführungsform des Schläfenabschnitts 36 im Querschnitt.

Aus Fig. 10 wird ersichtlich, dass der generelle Aufbau dem in Fig. 5 A dargestellten Aufbau der Bänderung 10 entsprechen kann. Der generelle Aufbau kann in anderen Ausführungsformen auch dem in Fig. 5 B und/oder C dargestellten Aufbau entsprechen oder derart sein, dass die Polsterschicht 22 und/oder die Verstärkungsschicht 24 auf den Stoffstrang 20, 21 aufgebracht und/oder integriert ist (nicht gezeigt).

In dieser Ausführungsform ist vorgesehen, dass der Schläfenabschnitt 36 zumindest abschnittsweise zumindest teilweise eine vorgeformte Kontur zur Bildung des Brillentunnels 40 aufweist. "Vorgeformte Kontur" bedeutet in diesem Zusammenhang, dass zumindest Teile der Bänderung 10 zumindest bereichsweise vorgeformt sind. Die Bänderung 10 ist in diesem Bereich nur begrenzt elastisch verformbar und gibt auch unter Zugkraft ihre vorgeformte Kontur nicht auf.

Es können alle Schichten der Bänderung 10 im Schläfenabschnitt 36 zumindest bereichsweise eine vorgeformte Kontur aufweisen oder nur einzelne Schichten. Alle möglichen Kombinationen sind denkbar.

Insbesondere ist die Bänderung 10 in dieser Ausführungsform derart vorgeformt, dass eine Wölbung ausgebildet ist, die den Brillentunnel 40 darstellt. Die Bänderung 10 kann beispielsweise derart ausgebildet sein, dass der Brillentunnel 40 zwischen der Bänderung 10 und dem (nicht dargestellten) Kopf 60 des Anwenders (siehe Fig. 10 A) und/oder innerhalb der Bänderung 10 ausgebildet ist (siehe Fig. 10 B).

In Fig. 10 A ist eine beispielhafte Ausführungsform gezeigt. Alle Schichten der Bänderung 10 weisen im Bereich des Schläfenabschnitts 36 eine vorgeformte Kontur auf. Der Brillentunnel 40 ist vom inneren Stoffstrang 21 zumindest teilweise begrenzt. Nach Anlage der Bänderung 10 am Kopf 60 befindet sich der Brillentunnel 40 zwischen dem innerem Stoffstrang 21 und dem (nicht gezeigten) Kopf 60 des Anwenders. In den Brillentunnel 40 kann sodann ein Brillenbügel 64 aufgenommen werden.

Die Bänderung 10 kann bei einer Anlage am Kopf 60 des Anwenders die in Fig. 2 dargestellten Geraden G1 und G2 zumindest bereichsweise kreuzen.

In dem Ausführungsbeispiel nach Fig. 10 A ist zwischen dem inneren Stoffstrang 21 der Bänderung 10 und einer zwischen den Kreuzungspunkten gezogenen Geraden (nicht gezeigt) die oben beschriebene Wölbung angeordnet, die den Brillentunnel 40 ausbildet.

In manchen Ausführungsformen kann die Bänderung 10 keinen inneren Stoffstrang 21 aufweisen, so dass die Wölbung, die den Brillentunnel 40 ausbildet, auch zwischen der Polsterschicht 22 und der zwischen den Kreuzungspunkten gezogenen Geraden verlaufen kann. In einer alternativen Ausführungsformen kann die Bänderung 10 weder inneren Stoffstrang 21 noch Polsterschicht 22 aufweisen, so dass die Wölbung, die den Brillentunnel 40 ausbildet, auch zwischen der Verstärkungsschicht 24 und der zwischen den Kreuzungspunkten gezogenen Geraden verlaufen kann. Die Wölbung beträgt mindestens 3 mm. Die vorgeformte Kontur ist derart eingerichtet und ausgebildet, dass der Brillentunnel 40 zwischen 3 mm und 30 mm hoch ausgebildet ist, bevorzugt zwischen 5 mm und 20 mm.

In alternativen Ausführungsbeispielen können auch einzelne und/oder mehrere Schichten der Bänderung 10 im Schläfenabschnitt 36 eine vorgeformte Kontur aufweisen während andere Schichten keine speziell vorgeformte Kontur aufweisen.

In Fig. 10 B ist eine beispielhafte Ausführungsform des Schläfenabschnitts 36 der erfindungsgemäßen Bänderung 10 gezeigt, bei der nur die Verstärkungsschicht 24 und der äußere Stoffstrang 20 eine vorgeformte Kontur aufweisen. Die Polsterschicht 22 und der innere Stoffstrang 21 sind in diesem Ausführungsbeispiel ohne eine vorgeformte Kontur ausgebildet.

Die Bänderung 10 kann bei einer Anlage am Kopf 60 des Anwenders die in Fig. 2 dargestellten Geraden G1 und G2 zumindest bereichsweise kreuzen.

In dieser Ausführungsform ist zwischen der Verstärkungsschicht 24 und der zwischen den Kreuzungspunkten gezogenen Geraden (nicht gezeigt) erfindungsgemäß die oben beschriebene Wölbung angeordnet, die den Brillentunnel 40 ausbildet. Die Wölbung beträgt mindestens 3 mm. Die vorgeformte Kontur ist derart eingerichtet und ausgebildet, dass der Brillentunnel 40 zwischen 3 mm und 30 mm hoch ausgebildet ist, bevorzugt zwischen 5 mm und 20 mm.

Da die Polsterschicht 22 und der innere Stoffstrang 21 in diesem Ausführungsbeispiel keine vorgeformte Kontur aufweisen, liegen die Polsterschicht 22 und der innere Stoffstrang auf einer Ebene mit der zwischen den Kreuzungspunkten gezogenen Geraden. Somit ist die Lücke, die den Brillentunnel 40 darstellt, zwischen der Verstärkungsschicht 24 und der Polsterschicht 22 ausgebildet. Verstärkungsschicht 24 und Polsterschicht 22 sind somit derart beabstandet voneinander angeordnet, dass der Brillentunnel 40 ausgebildet ist. Der Brillentunnel 40 ist zwischen 3 mm und 30 mm hoch ausgebildet ist, bevorzugt zwischen 5 mm und 20 mm.

In einem alternativen Ausführungsbeispiel können die Verstärkungsschicht 24 und die Polsterschicht 22 und der äußere Stoffstrang 20 eine vorgeformte Kontur aufweisen. In diesem Falle ist zwischen der Polsterschicht 22 und dem inneren Stoffstrang 21 eine Lücke ausgebildet, die den Brillentunnel 40 darstellt (nicht gezeigt).

Die erfindungsgemäße vorgeformte Kontur kann in Bezug auf den Aufbau nach Fig. 5C bedeuten, dass die Zwischenschicht, die gleichzeitig verstärkende und abpolsternde Eigenschaften aufweist und/oder der Stoffstrang 20, 21 im Schläfenabschnitt 36 eine vorgeformte Kontur aufweist, so dass Brillentunnel 40 ausgebildet ist (nicht gezeigt).

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Finzelmerkmale mit ein.

### Bezugszeichenliste

- 10: Bänderung
- 20: Äußerer Stoffstrang
- 21: Innerer Stoffstrang
- 22: Polsterschicht
- 23: Distanzpolster
- 24: Verstärkungsschicht
- 26: Verstärkendes Element
- 30: Seitenabschnitt
- 32: Zweiter Seitenabschnitt
- 34: Basisabschnitt
- 36: Schläfenabschnitt
- 40: Brillentunnel
- 60: Kopf
- 62: Schläfenbereich
- 64: Brillenbügel
- 70: Beatmungsgerät
- 80: Leitung
- 90: Patienteninterface / Atemmaske
- 92: Ansatzpunkt
- 94: Zweiter Ansatzpunkt
- 100: System

- B: Breite
- D: Dicke
- D22: Dicke der Polsterschicht
- D36/D36i: Dicke der Polsterschicht im Schläfenabschnitt
- G1: Gerade 1
- G2: Gerade 2
- L: Länge

- pra: Punkt der Ohrbasis (lat. *Praeaurale*)
- sa: Höchster Punkt der Ohrmuschel (lat. *Supeaurale*)
- sci: Höchster Punkt des Brauenoberrandes (lat. *Superciliare*)
- sn: Nasenwinkelpunkt (lat. *Subnasale*)

## Patentansprüche

1. Bänderung (10) für ein Patienteninterface (90), wobei die Bänderung (10) zumindest bereichsweise einen Brillentunnel (40) aufweist, der eingerichtet und ausgebildet ist, zumindest einen Brillenbügel (64) zumindest teilweise aufzunehmen, wobei die Bänderung (10) ferner einen inneren Stoffstrang (21), eine Verstärkungsschicht (24), eine Polsterschicht (22), einen äußeren Stoffstrang (20) und einen Schläfenabschnitt (36) umfasst, wobei der Schläfenabschnitt (36) zumindest bereichsweise den Brillentunnel (40) aufweist und derjenige Abschnitt der Bänderung (10) ist, der nach Anlage an einen Kopf (60) eines Anwenders über einen Schläfenbereich (62) des Kopfes (60) verläuft, wobei die Verstärkungsschicht (24) und/oder der äußere Stoffstrang (20) eine vorgeformte Kontur derart aufweisen, dass die Polsterschicht (22) und die Verstärkungsschicht (24) beabstandet voneinander sind und den Brillentunnel (40) ausbilden, wobei der innere Stoffstrang (21) und die Polsterschicht (22) im Schläfenabschnitt (36) keine vorgeformte Kontur aufweisen.

2. Bänderung (10) nach Anspruch 1, wobei der Brillentunnel (40) reversibel und/oder irreversibel ausgebildet ist.

3. Bänderung (10) nach Anspruch 2, wobei der reversibel ausgebildete Brillentunnel (40) durch die Einwirkung des Brillenbügels (64) und/oder durch einen lösbaren Zusatz zur Bänderung (10) ausgebildet ist.

4. Bänderung (10) nach Anspruch 2, wobei der irreversibel ausgebildete Brillentunnel (40) durch einen unlösbaren Zusatz zur Bänderung (10) und/oder durch eine zumindest teilweise Aussparung im inneren Stoffstrang (21) und/oder in der Verstärkungsschicht (24) und/oder in der Polsterschicht (22) und/oder im äußeren Stoffstrang (20) und/oder durch eine zumindest abschnittsweise zumindest teilweise Vorformung der Bänderung (10) ausgebildet ist.

5. Bänderung (10) nach zumindest einem der vorhergehenden Ansprüche, wobei in der Polsterschicht (22) und/oder der Verstärkungsschicht (24) und/oder dem Stoffstrang (20, 21) des Schläfenabschnitts (36) zumindest bereichsweise eine materialbedingte Schwachstelle ausgebildet ist, wobei die Schwachstelle derart ausgebildet und eingerichtet ist, dass durch Einwirkung des Brillenbügels (64) im Schläfenabschnitt (36) zumindest bereichsweise der Brillentunnel (40) reversibel ausgebildet wird.

6. Bänderung (10) nach Anspruch 5, wobei die Schwachstelle durch einen zumindest bereichsweise geringeren Härtegrad des Materials der Polsterschicht (22) und/oder der Verstärkungsschicht (24) und/oder des Stoffstranges (20, 21) ausgebildet ist.

7. Bänderung (10) nach zumindest einem der vorhergehenden Ansprüche, wobei die Polsterschicht (22) eine Dicke (D22) aufweist, die konstant ist oder zumindest bereichsweise nicht konstant ist.

8. Bänderung (10) nach zumindest einem der vorhergehenden Ansprüche, wobei die Polsterschicht (22) eine im Wesentlichen konstante Dicke (D22) aufweist und im Schläfenabschnitt (36) eine Dicke (D36) aufweist, die zumindest bereichsweise größer ist als die Dicke (D22), wobei durch Einwirkung des Brillenbügels (64) das Material der Polsterschicht (22) derart verdrängt wird, dass der Brillentunnel (40) reversibel ausgebildet wird.

9. Bänderung (10) nach zumindest einem der vorhergehenden Ansprüche, wobei die Bänderung (10) mindestens ein Distanzpolster (23) umfasst, das lösbar und/oder unlösbar mit der Bänderung (10) verbunden ist.

10. Bänderung (10) nach Anspruch 9, wobei das Distanzpolster (23) zumindest bereichsweise mit der Bänderung (10) verbunden ist, bevorzugt zumindest bereichsweise mit dem Schläfenabschnitt (36), wobei das Distanzpolster (23) im Schläfenabschnitt (36) derart angeordnet ist, dass der Brillentunnel (40) reversibel und/oder irreversibel ausgebildet wird.

11. Bänderung (10) nach zumindest einem der Ansprüche 9 oder 10, wobei das Distanzpolster (23) auf der Bänderung (10) befestigt ist und/oder in die Bänderung (10) integriert ist.

12. Bänderung (10) nach zumindest einem der vorhergehenden Ansprüche, wobei der Brillentunnel (40) durch eine zumindest teilweise Aussparung in der Polsterschicht (22) und/oder in der Verstärkungsschicht (24) und/oder in dem Stoffstrang (20, 21) ausgebildet ist.

13. Bänderung (10) nach zumindest einem der vorhergehenden Ansprüche, wobei der Brillentunnel (40) durch eine zumindest teilweise Aussparung der Polsterschicht (22) und/oder der Verstärkungsschicht (24) und/oder des Stoffstranges (20, 21) ausgebildet ist.

14. Bänderung (10) nach zumindest einem der vorhergehenden Ansprüche, wobei der Schläfenabschnitt (36) eine Länge (L) aufweist und die Aussparung in der Polsterschicht (22) und/oder in der Verstärkungsschicht (24) und/oder in dem Stoffstrang (20, 21) und/oder die Aussparung der Polsterschicht (22) und/oder der Verstärkungsschicht (24) und/oder des Stoffstranges (20, 21) 20 % bis 100 % der Länge (L) des Schläfenabschnitts (36), bevorzugt über 50 % bis 100 %, besonders bevorzugt 80 % der Länge (L) des Schläfenabschnitts (36) umfasst.

15. Bänderung (10) nach zumindest einem der vorhergehenden Ansprüche, wobei die Bänderung (10) zumindest abschnittsweise zumindest teilweise eine vorgeformte Kontur aufweist, die den Brillentunnel (40) irreversibel ausbildet.

16. Bänderung (10) nach zumindest einem der vorhergehenden Ansprüche, wobei der innere Stoffstrang (21) und/oder die Polsterschicht im Schläfenabschnitt (36) eine vorgeformte Kontur aufweisen.

17. Bänderung (10) nach zumindest einem der vorhergehenden Ansprüche, wobei die Bänderung (10) im Schläfenabschnitt (36) derart vorgeformt ist, dass die Bänderung (10) nach Anlage am Kopf (60) eines Anwenders zumindest teilweise derart beabstandet vom Kopf (60) des Anwenders ist, dass der Brillentunnel (40) ausgebildet ist.

18. Bänderung (10) nach zumindest einem der vorhergehenden Ansprüche, wobei der Brillentunnel (40) zwischen 3 mm und 30 mm hoch ausgebildet ist, bevorzugt zwischen 5 mm und 20 mm.

## Claims

1. A harness (10) for a patient interface (90), wherein the harness (10) has, at least in some regions, an eyeglass tunnel (40), which is designed and configured to at least partially receive at least one eyeglass arm (64), wherein the harness (10) also comprises an inner fabric strand (21), a reinforcing layer (24), a cushioning layer (22), an outer fabric strand (20), and a temple portion (36), wherein the temple portion (36) has the eyeglass tunnel (40) at least in some regions and is that portion of the harness (10) which runs over a temple region (62) of the head (60) after being placed on a head (60) of a user, wherein the reinforcing layer (24) and/or the outer fabric strand (20) have a preshaped contour such that the cushioning layer (22) and the reinforcing layer (24) are spaced apart from one another and form the eyeglass tunnel (40), wherein the inner fabric strand (21) and the cushioning layer (22) do not have a preshaped contour in the temple portion (36).

2. The harness (10) according to claim 1, wherein the eyeglass tunnel (40) is reversible and/or irreversible.

3. The harness (10) according to claim 2, wherein the reversible eyeglass tunnel (40) is formed by the action of the eyeglass arm (64) and/or by a detachable addition to the harness (10).

4. The harness (10) according to claim 2, wherein the irreversible eyeglass tunnel (40) is formed by a non-detachable addition to the harness (10) and/or by an at least partial cutout in the inner fabric strand (21) and/or in the reinforcing layer (24) and/or in the cushioning layer (22) and/or in the outer fabric strand (20) and/or by an at least partial deformation, at least in some portions, of the harness (10).

5. The harness (10) according to at least one of the preceding claims, wherein, at least in some regions, a material-dependent weak point is formed in the cushioning layer (22) and/or the reinforcing layer (24) and/or the fabric strand (20, 21) of the temple portion (36), wherein the weak point is designed and configured such that the eyeglass tunnel (40) is reversibly formed at least in some regions in the temple portion (36) through the action of the eyeglass arm (64).

6. The harness (10) according to claim 5, wherein the weak point is formed by a lower hardness, at least in some regions, of the material of the cushioning layer (22) and/or of the reinforcing layer (24) and/or of the fabric strand (20, 21).

7. The harness (10) according to at least one of the preceding claims, wherein the cushioning layer (22) has a thickness (D22) that is constant or that at least in some regions is not constant.

8. The harness (10) according to at least one of the preceding claims, wherein the cushioning layer (22) has a substantially constant thickness (D22) and in the temple portion (36) has a thickness (D36) that is at least in some regions greater than the thickness (D22), wherein the material of the cushioning layer (22) is displaced by the action of the eyeglass arm (64) such that the eyeglass tunnel (40) is reversibly formed.

9. The harness (10) according to at least one of the preceding claims, wherein the harness (10) comprises at least one spacer cushion (23), which is connected to the harness (10) detachably and/or non-detachably.

10. The harness (10) according to claim 9, wherein the spacer cushion (23) is connected to the harness (10) at least in some regions, preferably to the temple portion (36) in at least some regions, wherein the spacer cushion (23) is arranged in the temple portion (36) such that the eyeglass tunnel (40) is reversible and/or irreversible.

11. The harness (10) according to at least one of claims 9 or 10, wherein the spacer cushion (23) is fastened to the harness (10) and/or is integrated into the harness (10).

12. The harness (10) according to at least one of the preceding claims, wherein the eyeglass tunnel (40) is formed by an at least partial cutout in the cushioning layer (22) and/or in the reinforcing layer (24) and/or in the fabric strand (20, 21).

13. The harness (10) according to at least one of the preceding claims, wherein the eyeglass tunnel (40) is formed by an at least partial cutout of the cushioning layer (22) and/or the reinforcing layer (24) and/or the fabric strand (20, 21).

14. The harness (10) according to at least one of the preceding claims, wherein the temple portion (36) has a length (L) and the cutout in the cushioning layer (22) and/or in the reinforcing layer (24) and/or in the fabric strand (20, 21) and/or the cutout of the cushioning layer (22) and/or the reinforcing layer (24) and/or the fabric strand (20, 21) comprises 20% to 100% of the length (L) of the temple portion (36), preferably over 50% to 100%, particularly preferably 80% of the length (L) of the temple portion (36).

15. The harness (10) according to at least one of the preceding claims, wherein the harness (10) has, at least in some portions, at least partially a preshaped contour, which irreversibly forms the eyeglass tunnel (40).

16. The harness (10) according to at least one of the preceding claims, wherein the inner fabric strand (21) and/or the cushioning layer (22) and/or the reinforcing layer (24) and/or the outer fabric strand (20) have a preshaped contour in the temple portion (36).

17. The harness (10) according to at least one of the preceding claims, wherein the harness (10) is preshaped in the temple portion (36) such that, after being placed on the head (60) of a user, the harness (10) is at least partially spaced apart from the head (60) of the user such that the eyeglass tunnel (40) is formed.

18. The harness (10) according to at least one of the preceding claims, wherein the eyeglass tunnel (40) is between 3 mm and 30 mm high, preferably between 5 mm and 20 mm.

## Revendications

1. Agencement de brides (10) pour une interface de patient (90), dans lequel l'agencement de brides (10) présente au moins par endroits un tunnel à lunettes (40), lequel est configuré et formé pour recevoir au moins partiellement au moins une branche de lunettes (64), dans lequel l'agencement de brides (10) comporte en outre un cordon de tissu intérieur (21), une couche de renforcement (24), une couche de rembourrage (22), un cordon de tissu extérieur (20) et une section temporale (36), dans lequel la section temporale (36) présente au moins par endroits le tunnel à lunettes (40) et constitue la section de l'agencement de brides (10) s'étendant sur une région temporale (62) de la tête (60) après application sur une tête (60) d'un utilisateur, dans lequel la couche de renforcement (24) et/ou le cordon de tissu extérieur (20) présentent un contour préformé, de sorte que la couche de rembourrage (22) et la couche de renforcement (24) sont espacées l'une de l'autre et forment le tunnel à lunettes (40), dans lequel le cordon de tissu intérieur (21) et la couche de rembourrage (22) ne présentent pas de contour préformé dans la section temporale (36).

2. Agencement de brides (10) selon la revendication 1, dans lequel le tunnel à lunettes (40) est formé de façon réversible et/ou irréversible.

3. Agencement de brides (10) selon la revendication 2, dans lequel le tunnel à lunettes (40) formé de façon réversible est formé par l'action de la branche de lunettes (64) et/ou par une pièce complémentaire amovible de l'agencement de brides (10).

4. Agencement de brides (10) selon la revendication 2, dans lequel le tunnel à lunettes (40) formé de façon irréversible est formé par une pièce complémentaire inamovible de l'agencement de brides (10) et/ou par un évidement au moins partiel dans le cordon de tissu intérieur (21) et/ou dans la couche de renforcement (24) et/ou dans la couche de rembourrage (22) et/ou dans le cordon de tissu extérieur (20) et/ou par un préformage au moins partiel au moins par sections de l'agencement de brides (10).

5. Agencement de brides (10) selon l'une au moins des revendications précédentes, dans lequel une zone d'affaiblissement de matériau est formée au moins par endroits dans la couche de rembourrage (22) et/ou la couche de renforcement (24) et/ou le cordon de tissu (20, 21), dans lequel la zone d'affaiblissement est formée et configurée de telle façon que le tunnel à lunettes (40) est formé de façon réversible par l'action de la branche de lunettes (64) au moins par endroits dans la section temporale (36).

6. Agencement de brides (10) selon la revendication 5, dans lequel la zone d'affaiblissement est formée par un degré de dureté réduit au moins par endroits du matériau de la couche de rembourrage (22) et/ou de la couche de renforcement (24) et/ou du cordon de tissu (20, 21).

7. Agencement de brides (10) selon l'une au moins des revendications précédentes, dans lequel la couche de rembourrage (22) présente une épaisseur (D22) constante ou non constante au moins par endroits.

8. Agencement de brides (10) selon l'une au moins des revendications précédentes, dans lequel la couche de rembourrage (22) présente une épaisseur (D22) essentiellement constante et présente une épaisseur (D36) dans la section temporale (36) supérieure au moins par endroits à l'épaisseur (D22), dans lequel le matériau de la couche de rembourrage (22) est repoussé de telle manière par l'action de la branche de lunettes (64), que le tunnel à lunettes (40) est formé de façon réversible.

9. Agencement de brides (10) selon l'une au moins des revendications précédentes, dans lequel l'agencement de brides (10) comporte au moins un rembourrage d'espacement (23), lequel est relié de façon amovible et/ou inamovible à l'agencement de brides (10).

10. Agencement de brides (10) selon la revendication 9, dans lequel le rembourrage d'espacement (23) est relié au moins endroits à l'agencement de brides (10), de préférence au moins par endroits à la section temporale (36), dans lequel le rembourrage d'espacement (23) est disposé de telle manière dans la section temporale (36) que le tunnel à lunettes (40) est formé de façon réversible et/ou irréversible.

11. Agencement de brides (10) selon l'une au moins des revendications 9 ou 10, dans lequel le rembourrage d'espacement (23) est fixé à l'agencement de brides (10) et/ou intégré dans l'agencement de brides (10).

12. Agencement de brides (10) selon l'une au moins des revendications précédentes, dans lequel le tunnel à lunettes (40) est formé par un évidement au moins partiel dans la couche de rembourrage (22) et/ou dans la couche de renforcement (24) et/ou dans le cordon de tissu (20, 21).

13. Agencement de brides (10) selon l'une des revendications précédentes, dans lequel le tunnel à lunettes (40) est formé par un évidement au moins partiel de la couche de rembourrage (22) et/ou de la couche de renforcement (24) et/ou du cordon de tissu (20, 21).

14. Agencement de brides (10) selon l'une au moins des revendications précédentes, dans lequel la section temporale (36) présente une longueur (L) et l'évidement dans la couche de rembourrage (22) et/ou dans la couche de renforcement (24) et/ou dans le cordon de tissu (20, 21) et/ou l'évidement de la couche de rembourrage (22) et/ou de la couche de renforcement (24) et/ou du cordon de tissu (20, 21) mesure de 20 % à 100 % de la longueur (L) de la section temporale (36), de préférence de plus de 50 % à 100 %, de façon particulièrement préférée 80 % de la longueur (L) de la section temporale (36).

15. Agencement de brides (10) selon l'une au moins des revendications précédentes, dans lequel l'agencement de brides (10) présente au moins partiellement au moins par sections un contour préformé, lequel forme le tunnel à lunettes (40) de façon irréversible.

16. Agencement de brides (10) selon l'une au moins des revendications précédentes, dans lequel le cordon de tissu intérieur (21) et/ou la couche de rembourrage (22) et/ou la couche de renforcement (24) et/ou le cordon de tissu extérieur (20) présentent un contour préformé dans la section temporale (36).

17. Agencement de brides (10) selon l'une au moins des revendications précédentes, dans lequel l'agencement de brides (10) est préformé de telle façon dans la section temporale (36) que l'agencement de brides (10) est au moins partiellement espacé de la tête (60) de l'utilisateur après application sur la tête (60), de manière à former le tunnel à lunettes (40).

18. Agencement de brides (10) selon l'une au moins des revendications précédentes, dans lequel le tunnel à lunettes (40) présente une hauteur entre 3 mm et 30 mm, de préférence entre 5 mm et 20 mm.
